# EUROPEAN PATENT SPECIFICATION

(11) **EP 1 298 437 B1**
(45) Date of publication and mention of the grant of the patent: **08.08.2007**
(21) Application number: 02256651.7
(22) Date of filing: 25.09.2002
(51) Int. Cl.: G01N 33/52, C12Q 1/54

(54) **Colorimetric test device with reduced error**
Kolorimetrische Testvorrichtung mit geringerer Fehlerrate
Dispositif d'essai colorimétrique avec taux d'erreur réduit

(30) Priority: 26.09.2001 US 963243
(43) Date of publication of application: 02.04.2003
(73) Proprietor: LifeScan, Inc., Milpitas, CA 95035 (US)
(72) Inventor: Eyster, Curt R., San Jose, CA 95139 (US); Pugh, Jerry T., Mountain View, CA 94043 (US); Yu, Yeung S., St. Pleasanton, CA 94566 (US); Kim, Phuong, Hayward, CA 94544 (US)
(74) Representative: Mercer, Christopher Paul

(56) References cited:
- EP-A- 0 821 234
- US-A- 4 042 335
- US-A- 4 994 238
- US-A- 5 755 231
- US-A- 5 843 692

## Description

### Field of the Invention

The invention relates to test devices useful in colorimetric analyte determination. In particular, the invention provides a colorimetric test device in which at least a portion of the device's support is of a reflectivity that will not interfere with the meter's error detecting means thus facilitating low sample volume testing.

### Background of the Invention

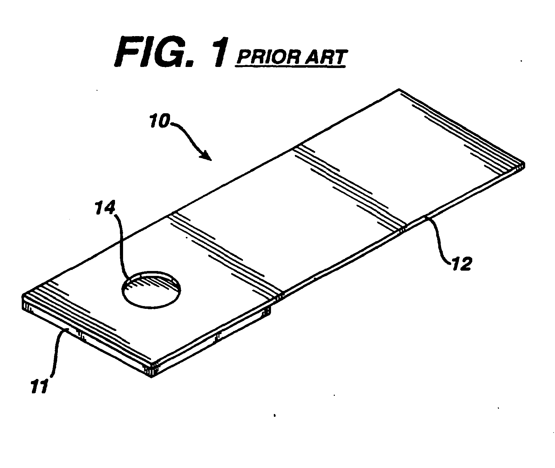

Colorimetric determination of analytes, meaning chemical and biochemical components, in body fluid samples is well known. For example, persons with diabetes place samples of their whole blood on test strips that are inserted into meters that determine blood glucose levels based on color changes induced in the test strip by the reaction of blood glucose and enzymes within the strip's test site. Fig 1 depicts a known test device 10 with aperture 14 in support 12 into which aperture 14 a sample is placed. Reagent pad 11, typically a hydrophilic material containing a suitable reagent, underlies aperture 14 for purposes of analyte testing. Light is reflected and measured on the opposite side of pad 11 to that which the sample is applied.

In performing colorimetric measurements, components such as red blood cells ("RBCs") that may interfere with the measurement must be filtered out. In devices, such as shown in Fig. 1, a filtering means is used to ensure that the fluid reaching the measurement side of the reagent pad is substantially free of RBCs. Further, it is desirable that the presence of RBCs and the background color due to their presence can be measured and corrected for by taking a measurement at a wavelength of approximately 700 nm.

Recently, colorimetric meters and testing devices useful therewith have been developed that use a smaller sample of blood than is required for testing in previously available systems (see US-A-5 843 692). Due to the smaller sample size, it is desirable to reduce the aperture on the testing device. However, it has been discovered that a portion of the light reflected back during measuring of RBCs at 700 nm may be due to the area of the testing device's support that surrounds the aperture. This may cause the reflectance at 700 nm to be higher than that for RBCs if the surrounding support material is more reflective than the RBCs. This result is disadvantageous because it adversely affects the meter's error detecting scheme. Thus, a need exists for a testing device useful in colorimeters that overcomes this disadvantage.

### Brief Description of the Drawings

FIG. 1 is a perspective view of a test device of the prior art.
Fig. 2 is a plan view of the bottom surface of one embodiment of the test device of the invention.
Fig. 3 is a plan view of the bottom surface of an alternative embodiment of the device of the invention.
Fig. 4 is a plan view of a preferred embodiment of the device of the invention.

### Detailed Description of the Invention and Preferred Embodiments

The present invention provides testing devices useful in colorimeteric measurements of analytes, as well as methods for their use and production, in which at least a portion of the device's support is of a reflectivity that will not interfere with the meter's error detecting means. The invention may find particular utility in the testing of small sample volumes, or of samples of less than about 5 µl. Thus, in one embodiment the invention provides a testing device comprising, or consisting essentially of, a support comprising or consisting essentially of, a top surface, a bottom surface and an aperture therethrough, wherein at least a portion of the bottom surface surrounding the aperture has a reflectivity of less than about 12 percent at between about 600 and 730 nm.

The test device of the invention may be useful in any of a wide variety of colorimeters. However, the invention may find particular utility in meters as described in United States Patent Nos. 4,935,346; 5,049,487; 5,304,468; 5,563,042; and 5,059,394. The invention may find further particular utility in colorimeters in such meters in which a sample of less than about 5 µl is used.

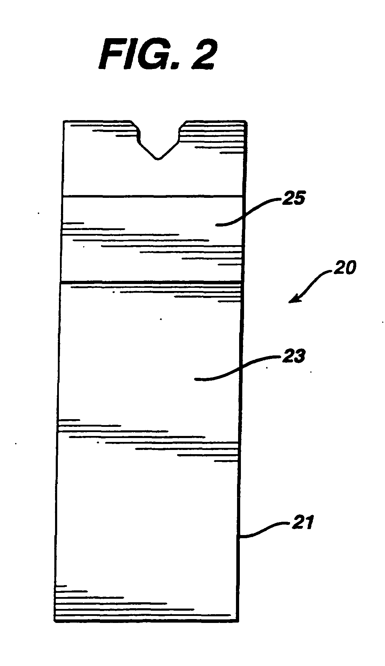

The test device of the invention may be of any shape, but preferably is a strip. In Fig. 2 is depicted a preferred embodiment of the invention. As shown, test strip 20 has support 21 with a top surface (not shown), bottom surface 23, and an aperture 24 therethrough (not shown). The aperture overlies reagent pad 25. The entire bottom surface 23 of support 21, and preferably the entirety of support 21 has a reflectivity of less than about 12 percent at between about 600 and 730 nm.

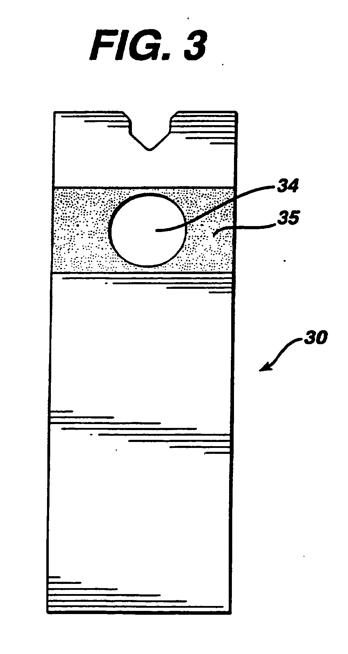

Fig. 3 depicts an alternative embodiment of the invention. In Fig. 3 is depicted test device 30 from which the reagent pad is removed. Area 35 surrounding aperture 34 is depicted, area 35 having a reflectance of less than about 12 percent at between about 600 and 730 nm. Preferably, area 35 is of dimensions such that, in combination with aperture 34, it corresponds to the entire optical viewing area for the meter with which the test device will be used. In yet another undepicted embodiment, the test strip is formed of a material that is transparent to light of the wavelengths used by the colorimeter.

In embodiments in which only a portion of the support is of the desired reflectivity, the material forming that portion of the support may itself be of the desired reflectivity or alternatively, the desired reflectivity may be achieved by coloring the area so that the desired reflectivity will be achieved. Coloring of the area may be achieved by any convenient means including, without limitation, printing a suitably colored ink onto the area, laminating a colored section on the area, or coloring the material from which the support will be made. For example, colorant may be added to polymer beads and extruded to form sheets of material from which the support may be formed.

The supports useful in the devices of the invention may be made of any material that is capable of supporting a reagent element and is sufficiently rigid to be inserted into or on a measuring device, such as a meter. Useful materials include, without limitation, thermoplastic materials. Preferably, the material is a polyolefin, such as a polyethylene or polypropylene, a polystyrene, a polyester, or combinations thereof. More preferably, the support is formed from a polystyrene.

The support may be of any dimensions suitable for use with a measurement device. Generally, the length dimensions are from about 15 to about 60 mm, the width dimensions are from about 5 to about 20 mm, and the thickness is about 0.1 to about 2.5 mm. Mounted on either the top or preferably, the bottom surface of the support is a reagent element that may be in any convenient form including, without limitation a membrane, pad, or the like. Typically and preferably, the reagent pad is a hydrophilic porous matrix with one or more suitable reagents impregnated into its pores. The reagents may be any reagent suitable for reacting with the target analyte to produce a compound that is characteristically absorptive at a wavelength other than a wavelength at which the assay medium substantially absorbs light. The reagent element is attached to the support by any convenient means for example by use of a non-reactive adhesive.

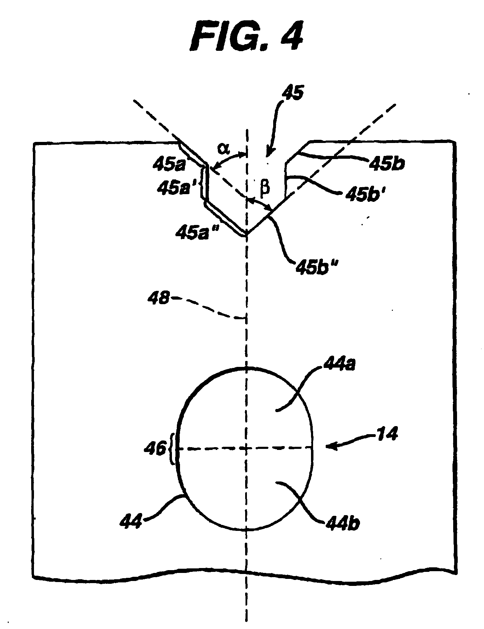

The aperture in the support over- or underlies the reagent element. The aperture may have any suitable configuration including, without limitation, circular, ovoid, elliptical, oblong, and the like. Preferably, the aperture is "obround" meaning that it is two halves of a circle extended apart by a straight midsection, as shown in Fig. 4. The aperture of Fig. 4 is defined by top and bottom half circles or arcs 44a and 44b and midsection 46. Arcs 44a and 44b each are defined by a base width in the range of about 3 to about 6 mm and an arc height of about 1.5 to about 3 mm. Midsection 46 is of the same width as the base width of arcs 44a and 44b and a height, along y axis 48, in the range of about 0.1 to about 0.2 mm. The total y axis tangent-to-tangent dimension for aperture 41 equals twice the arc diameter plus the length of midsection 46 and, thus, is about 3.1 to about 6.2 mm. Generally, the sample volume applied to the aperture is about 5 to about 50 µl and in the preferred embodiment is about 5 µl or less.

The device of the invention preferably includes an alignment notch at one end for aligning of the device in the measurement instrument with which it will be used. In the preferred embodiment of the device, and as shown in Fig. 4, the notch has opposing, mirror image edges that are in substantially parallel relationship to each other and with centerline 48. In a more preferred embodiment, notch 45 has three pairs of opposing edges 45a, 45b, 45a' and 45b', and 45a" and 45b". Edge segments 45a and 45b are each set at an angle α that preferably ranges from 30 to 60° and the segments have lengths of about 0.5 to about 2.0 mm. The distal edges of 45a and 45b extend laterally from centerline 38 for a distance, preferably about 2.0 to about 3.0 mm. The distal ends each extend laterally from centerline 48 a distance of about 1.0 to about 2.0 mm.

Segments 45a' and 45b' extend downwardly from the proximal ends of 45a and 45b respectively and are substantially parallel to centerline 48. Segments 45a' and 45b' have lengths preferably about 0.5 to about 2.0 mm. Segments 45a" and 45b" extend inwardly from the proximal ends of 45a' and 45b' each forming an angle β with centerline 48 that ranges from about 30 to about 60 °. The proximal ends of 45a" and 45b" intersect at centerline 48.

The test device of the invention may be used with any colorimetric instrument, such as a meter, adapted and suitable for measuring a targeted analyte in a fluid sample including, without limitation, a physiological or biological fluid sample such as blood, interstitial fluid, or the like. The meter optionally, but typically and preferably, includes a test device holder into which the device is inserted and an alignment pin either in the device holder or a test device receptacle area. The alignment notch of the test device has a configuration for engagement with the alignment pin to ensure proper alignment of the device upon insertion. Additionally, the notch-pin engagement maintains the test device in a substantially motionless position with respect to the alignment pin when the device is operatively engaged within the device holder or meter.

A variety of analytes may be detected and their concentrations determined using the test device of the invention. Illustrative analytes include, without limitation, glucose, cholesterol, lactate, alcohol, and the like. In a method for use of the device of the invention, the test device is provided for receiving a fluid sample. Prior to, or after, insertion of the device into a suitable measuring instrument, for example a meter, a quantity of the fluid is applied or introduced to the device's aperture by any convenient method including, without limitation, deposition, injection, wicking, or the like. The sample volume applied to the aperture is about 5 to about 50 µl, preferably about 5 µl or less. The sample is allowed to react with the reagent of the reagent element to produce a detectable product that is then related to the amount of analyte in the sample by the measurement instrument. Automated meters for detecting and measuring the product for use with colorimetric assays are well known in the art as for example disclosed in U.S. Patent No. 5,059,394.

In another embodiment, a kit is provided comprising, a measurement instrument and at least one test device of the invention. The kit also may include sampling accessories including, without limitation, a blood letting device, such a lancet, a control solution, and the like, and combinations thereof.

The invention will be clarified further by consideration of the following, nonlimiting examples.

### Examples

### Example 1

The area of the bottom surfaces surrounding the aperture of several polystyrene test strips was colored as follows: gray test strip marked with black; gray test strip marked with blue; and gray test strip marked with red. Additionally, transparent, white and black test strips were formed.

The strips were formed by fashioning a polymer sheet with the color band on the back side of the sample application port into a card. Adhesive tape was applied over the back side of the sample application port and a reagent impregnated membrane previously calibrated with the standard test strip design of gray polystyrene plastic was affixed to the adhesive. The resulting card was then cut into strips for testing. A 5 µL sample of 25 % hematocrit blood spiked to 50 mg/dL glucose was applied to the aperture of the strip and the progress of the chemical reaction was monitored using a ONE TOUCH® Basic meter. After approximately 45 secs. either a blood glucose reading, a control solution reading, or an insufficient blood error was reported by the meter. The results of the testing demonstrated that the transparent, black and black-marked gray strips, having reflectivities of less about 12 percent at approximately 600 to 730 nm, produced one or less error messages.

### Example 2

A wax transfer-type computer printer ALPS MD1000 thermal transfer printer was used to print a 0.5 inch wide color band over the aperture area on the bottom surface of gray polystyrene test strips. Strips having blue and black color bands and reflectivities of less than 12 percent at approximately 600 to 730 nm were produced and tested for control solution errors as in Example 1. Neither type of strip produced control solution error messages.

## Claims

1. A test device, comprising:
a.) a support member (21) defining a longitudinal axis and comprising:
i.) a top surface, a bottom surface (23) and an aperture (24) therethrough;
ii.) a distal edge substantially transverse to the longitudinal axis and configured for insertion into a measurement instrument; and
iii.) alignment notch (45) formed in the distal edge for engagement with an alignment member of the measuring device and comprising opposing edges wherein at least a portion of the opposing edges is in substantially parallel relation to the longitudinal axis; and
b.) a reagent pad (25) fixed to the support member and covering the aperture, the reagent material selected for reacting with at least one analyte,
**characterised by** at least a portion of the bottom surface surrounding the aperture having reflectivity of less than 12 percent at between 600 and 730 nm.

2. The test device of claim 1, wherein the opposing edges comprise at least three portions wherein two of the three portions of the opposing edges are in angular relation to the longitudinal axis.

3. The test device of claim 1 or claim 2, wherein the portion of the opposing edges in substantially parallel relation is located in between the two portions of the opposing edges in angular relation to the longitudinal axis.

4. The device of any one of claims 1 to 3, wherein substantially the entire bottom surface has a reflectivity of less than 12 percent at between 600 and 730 nm.

5. The device of any one of claims 1 to 4, wherein the aperture holds a fluid volume of less than or equal to 5 µl.

6. The device of any one of claims 1 to 5, wherein the aperture is obround-shaped.

7. A system for measuring the concentration of at least one analyte in a fluid, comprising:
a.) at least one test device corresponding to any one of claims 1 to 6 ; and
b.) a colorimeter.

## Patentansprüche

1. Testvorrichtung, mit:
a) einem Trägerbauteil (21) mit einer longitudinale Achse, mit:
i) einer oberen Oberfläche, einer unteren Oberfläche (23) und eine durch die obere Oberfläche und die untere Oberfläche (23) gebildete Apertur (24);
ii) einer im wesentlichen transversal zu der longitudinalen Achse distalen Kante, welche für eine Einführung in einem Meßinstrument konfiguriert ist; und
iii) einer Ausricht-Aussparung (45), welche in der distalen Kante gebildet ist, um mit einem Ausricht-Bauteil des Meßinstruments zusammen zu wirken, mit einander gegenüberliegenden Kanten, wobei wenigstens ein Bereich der einander gegenüberliegenden Kanten im wesentlichen parallel zu der longitudinalen Achse ist; und
b) einer Reagenzfläche (25), welche an dem Trägerbauteil (21) befestigt ist und die Apertur bedeckt, wobei das Reagenzmaterial derart gewählt ist, daß es mit wenigstens einem Analyten reagiert, **dadurch gekennzeichnet, daß** wenigstens ein Bereich der unteren Oberfläche, welcher die Apertur umgibt, zwischen 600 und 730nm eine Reflektivität von weniger als 12 % aufweist.

2. Testvorrichtung nach Anspruch 1, **dadurch gekennzeichnet, daß** die einander gegenüberliegenden Kanten wenigstens drei Bereiche aufweisen, wobei zwei der drei Bereiche der einander gegenüberliegenden Kanten gewinkelt zu der longitudinalen Achse sind.

3. Testvorrichtung nach Anspruch 1 oder 2, **dadurch gekennzeichnet, daß** der Bereich der einander gegenüberliegenden Kanten, welcher im wesentlichen parallel zu der longitudinalen Achse ist, zwischen den zwei Bereichen der einander gegenüberliegenden Kanten angeordnet ist, welche gewinkelt zu der longitudinalen Achse sind.

4. Testvorrichtung nach einem der vorangehenden Ansprüche, **dadurch gekennzeichnet, daß** im wesentlichen die gesamte untere Oberfläche zwischen 600 und 730nm eine Reflektivität von weniger als 12% aufweist.

5. Testvorrichtung nach einem der vorangehenden Ansprüche, **dadurch gekennzeichnet, daß** die Apertur ein Fluidvolumen von weniger oder gleich 5 µl aufweist.

6. Testvorrichtung nach einem der vorangehenden Ansprüche, **dadurch gekennzeichnet, daß** die Apertur eine "obround" ähnliche Form aufweist.

7. System zum Messen der Konzentration von wenigstens einem Analyten in einem Fluid, mit:
a) wenigstens einer Testvorrichtung nach einem der vorangehenden Ansprüche; und
b) einem Kolorimeter.

## Revendications

1. Dispositif d'essai, comprenant :
a) un élément de support (21) définissant un axe longitudinal et comprenant :
i) une surface supérieure, une surface inférieure (23) et une ouverture (24) à travers ;
ii) un bord distal sensiblement transversal à l'axe longitudinal et configuré pour une insertion dans un instrument de mesure ; et
iii) une encoche d'alignement (45) formée dans le bord distal pour venir en prise avec un élément d'alignement du dispositif de mesure et comprenant des bords opposés dans lesquels au moins une partie des bords opposés se trouve dans une relation sensiblement parallèle à l'axe longitudinal ; et
b) un tampon de réactif (25) fixé à l'élément de support et couvrant l'ouverture, le matériau de réactif étant sélectionné pour réagir avec au moins une substance à analyser,
**caractérisé par le fait qu'**une partie au moins de la surface inférieure qui entoure l'ouverture, présente une réflectivité inférieure à 12 pour cent entre 600 nm et 730 nm.

2. Dispositif d'essai selon la revendication 1, dans lequel les bords opposés comprennent au moins trois parties dans lesquelles deux des trois parties des bords opposés présentent une relation angulaire par rapport à l'axe longitudinal.

3. Dispositif d'essai selon l'une quelconque des revendications 1 ou-2, dans lequel la partie des bords opposés en relation sensiblement parallèle, est située entre les deux parties des bords opposés qui présentent une relation angulaire par rapport à l'axe longitudinal.

4. Dispositif selon l'une quelconque des revendications 1 à 3, dans lequel sensiblement toute la surface inférieure présente une réflectivité inférieure à 12 pour cent entre 600 nm et 730 nm.

5. Dispositif selon l'une quelconque des revendications 1 à 4, dans lequel l'ouverture contient un volume de fluide inférieur ou égal à 5 µl.

6. Dispositif selon l'une quelconque des revendications 1 à 5, dans lequel l'ouverture présente une forme arrondie.

7. Système de mesure de la concentration d'au moins une substance à analyser dans un fluide, comprenant :
a) au moins un dispositif d'essai correspondant à l'une quelconque des revendications 1 à 6 ; et
b) un colorimètre.
